# EUROPEAN PATENT SPECIFICATION

(11) **EP 3 514 227 B1**
(45) Date of publication and mention of the grant of the patent: **10.06.2020**
(21) Application number: 16916200.5
(22) Date of filing: 13.09.2016
(51) Int. Cl.: C12N 5/071, A61L 27/00, C12M 1/00, C12N 5/09, A61L 27/38, C12M 3/06, C12N 5/00

(54) **METHOD FOR PRODUCING THREE-DIMENSIONAL CELL STRUCTURE AND SUPPORT USED FOR SAME**
VERFAHREN ZUR HERSTELLUNG EINER DREIDIMENSIONALEN ZELLSTRUKTUR UND TRÄGER DAFÜR
PROCÉDÉ DE PRODUCTION D'UNE STRUCTURE CELLULAIRE TRIDIMENSIONNELLE, ET SUPPORT SERVANT AUDIT PROCÉDÉ DE PRODUCTION D'UNE STRUCTURE CELLULAIRE TRIDIMENSIONNELLE

(43) Date of publication of application: 24.07.2019
(73) Proprietor: Ono, Jiro, Kitamoto-shi, Saitama 364-0007 (JP)
(72) Inventor: Ono, Jiro, Kitamoto-shi, Saitama 364-0007 (JP)
(74) Representative: Forrest, Stuart
(86) International application number: PCT/JP2016/076987
(87) International publication number: WO 2018/051415

(56) References cited:
- EP-A1- 2 130 910
- WO-A1-2008/123614
- WO-A1-2008/143149
- WO-A1-2010/069080

## Description

### FIELD OF INVENTION

This invention relates generally to the field of fabricating of three-dimensional engineered tissue using cell aggregates, especially the method of fabricating three-dimensional engineered tissue using the net-like shape or mesh-like supports.

### BACKGROUND

As world population increase as well as prolonged human lifetime span, needs, desires and demands for medical treatments are only elevating. In recent years, a new approach, Tissue Engineering, which utilize living cell, is spotlighted. Ultimate goal of Tissue Engineering is to fabricate artificial tissue and organ for transplantation to human body. Artificial tissue is fabricated part of the body which express particular functionality when transplanted to human body. However, transplantation to human body requires number of approving process which comes with long span activities.
Aside from human transplantation, another promising application is to use engineered tissue as research specimen, which include toxicity screening, drug effect judgement, pathology judgement, developmental research, etc. By fabricating engineered tissue with only human cells and use them for experiment, it will be possible to reproduce or mimic in vivo environment of human body. This leads to higher drug discovery research, personalized medication diagnosis and development observations, etc.
Especially in the field of cancer, medication diagnosis and effect prediction is always difficult one. This technology may enable better prediction when text specimen is made with patient own cancer cells and tested number of different drugs ex-vivo, or outside of the body, prior to actual medications.

There are two type cells, one is floating cell and another is adherent cell which required scaffold. Floating cell includes blood cells and immune system, and adherent cell are organ, skin and bone cells. Adherent cell shall not be able to survive in long period when floating in cell suspension, and it is necessary to let them adhere to any scaffold such as dish or gel and such so that they can survive and proliferate. When Adherent cell is located in non-adherent environment, which means there are nothing to adhere to, cells seek for scaffold and start adhering each other and consequently forms large cell aggregate, which is three-dimensional cellular structure. This phenomenon is widely known and reported in reference non-patent articles #1 ∼#6. Non-Patent Article 1 describe this cell aggregating phenomenon is known as old as 1960's. Especially Non-Patent Article #6 clarify the idea using cell aggregate as "Building Block" which indicate that diversified type of cell could be used.

Patent Document #1 describe method of fabricating Tissue plug from only living cell with desired shape without support. In detail, this process use chamber which possess micro-pore only at the bottom which enable medium to pass through, and apply just enough mediums inside of chamber so that part of cell aggregate are exposed to air, use in excess of medium of total chamber volume for maturation of cellular structure.

Also, there are number of method of making three-dimensional cellular structure, and Patent Document #2 describes dispensing method which dispense cell aggregate onto plane surface from printer nozzle, whereas Patented Document #3 shows process needle array process which penetrate through Spheroids with needs. Needle Array process use support consists of number of needles mounted on base, which needles penetrate number of spheroids for alignment, and remove needles after the maturation to obtain 3D cellular structure. Furthermore, Patent Document #4 shows process of making 3D Cellular structure by accumulating multiple plane cultured cell sheets which was made on permeable sheet.
EP 2130910 discloses a method for arranging various cells as cell clusters in an arbitrary three-dimensional space and producing a three dimensional structure of a desired shape constituted exclusively by cells. The document also discloses a support provided with a substrate and a thread or needle-shaped material that penetrates the substrate and cell clusters for positioning cell clusters in arbitrary space. The support is provided with a sheet that can be removed as necessary for covering the substrate. A method for using the support structure to position cell clusters in an arbitrary space and a method for the production of three-dimensional cell structures is also disclosed.
WO2010/069080 discloses a filter apparatus comprising a filter having first and second opposed surfaces, wherein a first reservoir is positioned adjacent with the first surface and in communication with the first surface, and a first inlet-outlet port is in communication with the first reservoir and spaced from the first surface. A second reservoir is positioned adjacent the second surface, and in communication with the second surface, and a second inlet-outlet port is in communication with the second reservoir and spaced from the second surface. The document also discloses a filter plate system comprising a reservoir plate, wherein the reservoir plate comprises at least one reservoir well. The filter plate system further comprises a strainer plate. The strainer plate comprises at least one strainer well, which is removably receivable in the reservoir well. The strainer well comprises at least one mesh wall portion.

### THE PRIOR ART LITERATURE

### Patent Document

Patent Document #1: JP-P4122280
Patent Document #2: US 8,852,932
Patent Document #3: WO2017150366 A1 (PCT/JP2017/007029)
Patent Document #4: WO2005/047496

Non-Patent Article #1: PLOS ONE, Journal. Pone. 0136681, "Scaffold-Free Tubular Tissues Created by a Bio-3D printer Undergo Remolding and Endothelialization when Implanted in Rat Aortae", Manabu Itoh et al, September 1, 2015
Non-Patent Article #2: Gordon R, Goel NS, Steinberg MS, Wiseman LL. A rheologic a1 mechanism sufficient to explain the kinetics of cell sorting. J Theor Biol. 1972; 37:43-73. [PubMed: 4652421]
Non-Patent Article #3 : Jakab K, Damon B, Marga F, Doaga O, Mironov V, Kosztin I , Markwald R, Forgacs G. Relating cell and tissue mechanics: implications and applications. Dev. Dyn. 2008; 237:2438-2449. [PubMed: 1872 9216]
Non-Patent Article #4 : Jakab K, Neagu A, Mironov V, Markwald RR, Forgacs G. Engineering biological structures of prescribed shape using self-assembling multicellular systems. Proc Natl Acad Sci U S A. 2004; 101:2864 -2869. [PubMed: 14981244]
Non-Patent Article #5 : Perez-Pomares JM, Foty RA. Tissue fusion and cell sorting in embryonic development and disease:biomedical implications. Bioessays. 2006: 28:809-821. [PubMed: 16927301]
Non-Patent Article #6 : Organ printing: Tissue spheroids as building blocks" Biomaterials. Vladimir Mironov, Richard P. Visconti, Vladimir Kasynocv, Gabor Forgacs, Christopher J. Drake, and Roger R. Markwald, 2009 April ; 30(12):2164-2174. doi: 10.1016

### SUMMARY Of INVENTION

### Problems to be solved by the invention

Most of dispensing method for three-dimensional Cellular structure fabrication such as described in Patent Document #2 are either a process which dispense Bio-Ink , which consists of spheroid and connecting material such as Hydrogel or collagen, onto flat surface, or a process injecting spheroids into pre-build scaffold made with shape maintaining material which can be Hydrogel or Collagen. Downside of this method is that Scaffold material block oxygen and nutrients delivery to cells. Furthermore, this patent documents claim to "arrange a plurality of cell aggregates according to a pattern such that each of the cell aggregates contacts at least one other cell aggregate," which requires "arranging according to a pattern". Also, as shape of these types of three-dimensional cellular structure depends largely on shape retaining capability of scaffold materials, there are limitation in size and shape (especially height direction). Furthermore, Scaffold will remain inside of three-dimensional Cellular structure, that leaves possibility of potential adverse effect against cell, which cause additional evaluation or approval process when transplanted to human patient.

Patent Document #3 describe about Needle Array process which has predetermined separation distance of each needle, several conditions toward cell aggregate are required, such as, cell aggregate has to be sphere shape in general, soft enough that needle can pierce through as well as hard enough that cell aggregate remain at the same position on the needle, and cell aggregate size has to be within certain range so that they shall be contacting each other on the needle. As this process pick an place cell aggregate one at a time, total operation time shall be considerably long. Furthermore, precise needle location is essential to this process and that requires precise manufacturing, which lead to higher manufacturing cost as well as special equipment to handle needle array.

The present invention solves above mentioned problems and aim to provide better and easier method of fabricating three-dimensional cellular structure and supporting device ding the same.

### The method to solve problems

The present invention provides a method of fabricating a cellular structure, such as a three dimensional cellular structure. The method comprises a step of feeding cell aggregate onto thread or needle shaped members, said member forming a net shaped space, such as a meshed space, by said thread or needle shaped members, a step of layering a support with feed cell aggregate, and a step of removing said threads or needles from fused cellular structure, wherein the support comprises multiple frames and multiple thread or needle shaped members which form a net shaped space surrounded by the multiple frames; wherein said net shaped space can hold cell aggregates.

Also, the present invention provides a method of fabricating a cellular structure, the method comprising a step of layering supports having a net shaped space, such as a meshed space, formed by thread or needle shaped members, a step of feeding cell aggregate onto the said layered support, and a step of removing said thread or needle shape members from fused cellular structure, wherein the support comprises multiple frames and multiple thread or needle shaped members which form a net shaped space surrounded by the multiple frames; wherein said net shaped space can hold cell aggregates.

The present invention also provides a method of fabricating a cellular structure, the method comprising a step of layering supports, said support having a thread or needle shaped member, said member provide a boundary of the arbitrary cellular structure shape, a step of feeding a cell aggregate onto support; and a step of removing said threads or needles from a fused cellular structure, wherein the support comprises multiple frames and multiple thread or needle shaped members which form a net shaped space surrounded by the multiple frames, wherein said net shaped space can hold cell aggregates

The present invention also provides a method of fabricating a cellular structure utilizing automated dispensing equipment, the method comprising a step of preparing support comprising multiple frames and multiple thread or needle shaped members which form net shaped space surrounded by the multiple frames, wherein said net shaped space can hold cell aggregates, a step of holding cell aggregates being fed from said automated dispensing equipment within said net shaped space after, and a step of removing thread or needle shaped member from fused cell aggregate.

A method of making a support described in present invention comprising forming a mask layer onto thin sheet member, and forming thread or needle shaped member by etching exposed area of said thin sheet member is also disclosed. Furthermore, a method of making support described in present invention may comprise forming thread or needle shaped members by bonding wire.

### ADVANTAGEOUS EFFECT OF PRESENT INVENTION

The present invention provides easier fabrication method compared to existing methods, by enabling to remove thread or needle shaped member one by one from cell aggregate after said cell aggregates fused together, wherein said aggregate filled into the space which is formed by thread or needle shaped members.

### BRIEF DESCRIPTION OF THE DRAWINGS

[Fig. 1] Perspective view of first embodiment of support for three-dimensional cellular structure fabrication.
[Fig. 2] Plan view of said support shown in Fig. 1.
[Fig. 3] Rear view of said support shown in Fig. 1.
[Fig. 4] Perspective view of first embodiment of supports being layered.
[Fig. 5] Example of how first embodiment of support can be disassembled.
[Fig. 6] Schematic flow of fabrication process of three-dimensional cellular structure concerning the first embodiment of present invention.
[Fig. 7] Shows how cell aggregate are being fed to support.
[Fig. 8] Explain how cell aggregate are being dispensed by dispenser.
[Fig. 9] Shows layering of supports which cell aggregate are already dispensed.
[Fig. 10] (A) shows schematic perspective view of three-dimensional cellular structure after frame of support, and (B) shows schematic perspective view of three-dimensional cellular structure after thread shaped member are removed.
[Fig. 11] One modification example of present invention, which shows dispensing after multiple supports are being layered.
[Fig. 12] Perspective view showing composition of support concerning the second embodiment of present invention.
[Fig. 12A] One of modification example of second embodiment of present invention.
[Fig 13] Shows how cell aggregate are fed onto supports concerning second embodiment of present invention.
[Fig. 13A] Shows example of how thread members are being removed after cutting supports.
[Fig. 14] Schematic perspective view of three-dimensional cellular structure fabricated using support concerning second embodiment of present invention.
[Fig. 15] Shows composition of support concerning third embodiment of present invention.
[Fig. 16] Schematic perspective view of three-dimensional cellular structure fabricated using support concerning third embodiment of present invention.
[Fig. 17] Shows modification of the third embodiment of present invention.
[Fig. 18] Explains a fabrication method of three-dimensional cellular structure.
[Fig. 19] Explain fabrication method of three-dimensional cellular structure by the fifth embodiment of present invention.
[Fig. 19A] Explain fabrication method of three-dimensional cellular structure by the sixth embodiment of present invention.
[Fig. 20] Sectional view of support when manufactured by semiconductor manufacturing process.
[Fig. 20A] Shows sectional view of support when formed by bonding wire.
[Fig. 21] Shows one example of manufacturing equipment for three-dimensional cellular structure concerning embodiment of present invention.
[Fig. 22] Shows manufacturing process of support by 3D Printer concerning the sixth embodiment of present invention.

### DESCRIPTION OF THE INVENTION

Next, the method of fabricating three-dimensional cellular structure as embodiment of present invention comprise; frame, support with string member (thread or needle shaped) which form net shaped or matrix shaped space inside of the frame, filling said net shaped space with cell aggregate (Spheroid), remove said string member from fused cellular aggregates to fabricate three-dimensional cellular structure.

### EMBODIMENTS

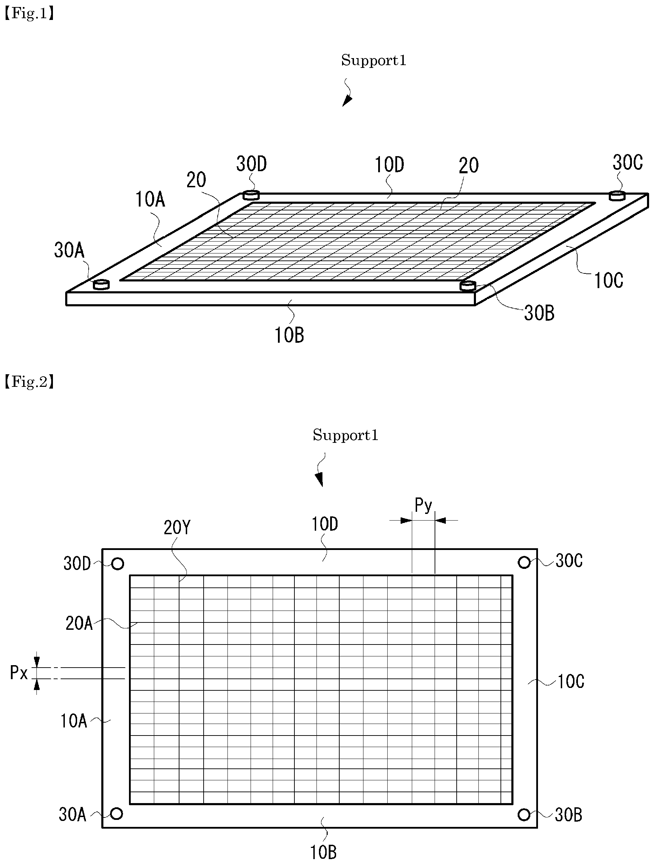

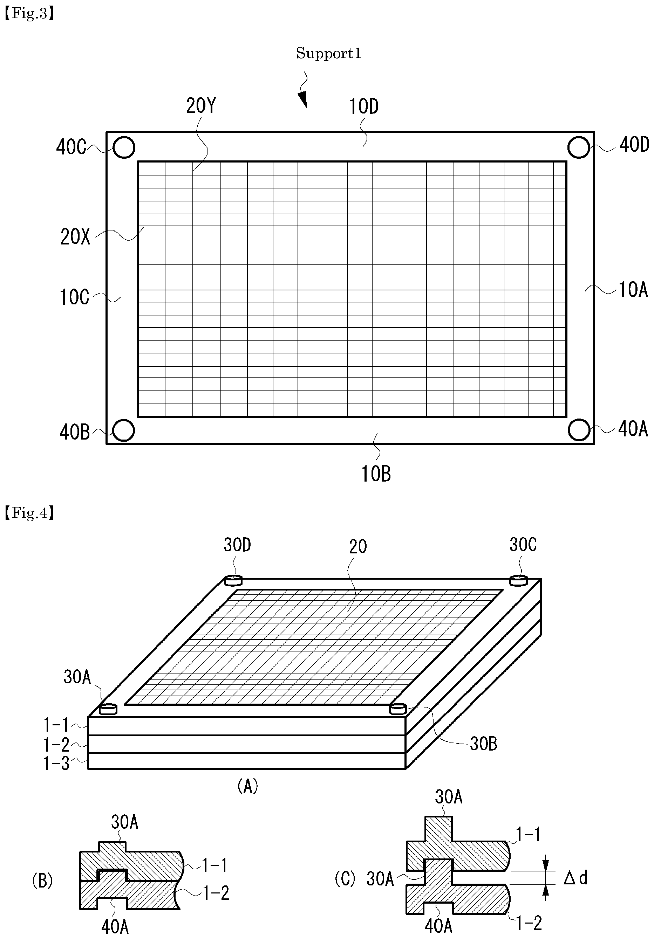

Below are explanation of embodiments. Fig. 1 is perspective view of first embodiment of support for three-dimensional cellular structure fabrication. Fig. 2 is plan view of said support shown in Fig. 1. Fig. 3 is Rear view of said support shown in Fig. 1. Support 1 consists of frame10A, frame10B, frame10C and frame 10D forming four side of frame (called hereinafter frame10 as a whole frame), multiple string member forming net or matrix shaped space inside of frame 10, alignment measure to align each frame when layered.

Frame10 can be made of arbitrary materials, such as resin. Also, size of frame10 can be any size, as an example one side length can be 3-4cm and thickness can be 0.1-1.0mm when cell aggregate size is around 200um.

String Member20 exists in direction of X and Y inside of frame10. String member20 can be made with materials such as stainless steel, nylon, polyester, and diameter can be 10um as one example. Preferably string member20 is coated with non-adhesive material such as P-HEMA, and sterilized with ethanol. String member20X existing in x direction connect to frame10A (by adhesive or such, for example) while another end connected to frame10C. String member20Y existing in Y direction connect to frame10A (by adhesive or such, for example) while another end connected to frame10C. Multiple String member20X in x direction are placed at uniform gap distance Px, and multiple String member20Y in y direction are placed at uniform gap distance Py, consequently both members forming rectangular shaped spaces of Px and Py inside of frame10. Gap distance Px and Py can be 0.1mm as an example. Also, ratio of Px and Py can be determined by aspect ratio of frame10 (for example, Px:Py=4:. When aspect ratio of frame10 is 4:3) but not limited to this ratio.

As explained later, cell aggregates are dispensed onto string member20 of support 1, and dispensed cell aggregate has to be held at net shaped space formed by string member20. Thus, the size of net shaped space formed by string member20 (Px, Py) has to be smaller than the size of cell aggregates to be dispensed. However, uniformity of cell aggregate size is not necessary. It should be noted that simply smallest size of cell aggregate Dmin needed to be larger than Px, Py. (Dmin>Px, Dmin>Py)

As one of good examples, in order to fabricate three-dimensional cellular structure, identical shaped number of support1 are layered as shown in Fig. 4(A). Support1 has alignment measure30 to align each support when layered. Alignment measure30 can be, but not limited to, dovetail fitting member, fastening by bolts, and other engaging measures. Figure shows cylindrical projections30A, projection30B, projection30C and projection30D are formed on each corner of support1, and recess40A, recess40B, recess40C and recess40D are formed on back side of supprtl which shall engaged to projections30A, projection30B, projection30C and projection respectively. Utilizing these projection30 and recess40, layering multiple support can be easily done. Fig. 4 shows an example of three supports, suooprt1-1, support1-2 and support1-3 are being layered. By selecting height of projection30 and/or depth of recess40, arbitrary space between supports can be obtained. Fig.4(B) shows projection30 height is smaller than the depth of recess40, which consequently forming space Δd between support1-1 and support1-2. Δd can be selected depending on thickness of support1 and/or size of cell aggregate to be dispensed.

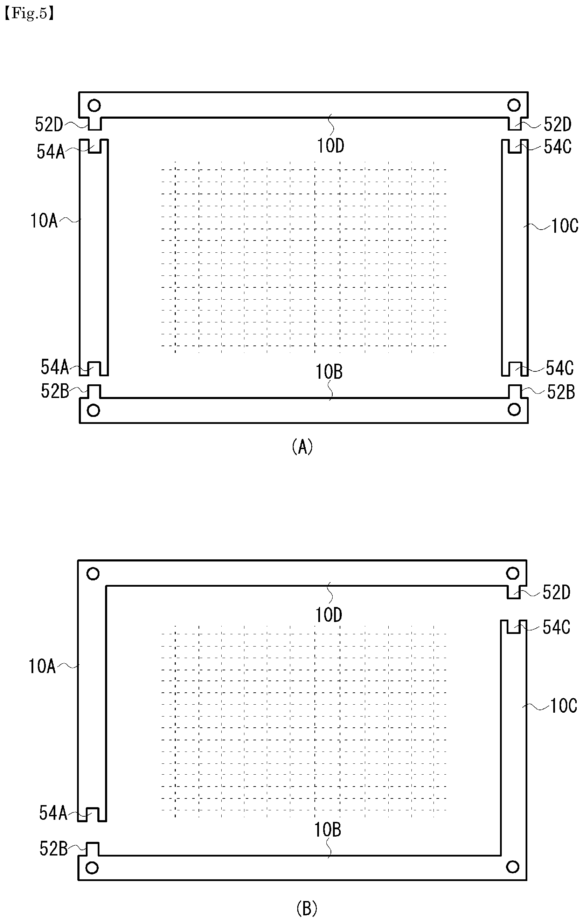

Preferably one of the frame out of multiple frames is detachable from other frames. As explained later, this makes removal of string member20 from fused sell aggregate easier after cell aggregate fused together. Fig. 5(A) shows one example how frame is separated from the others. Frame10A has recess54A on both ends, frame10C has recess52B on both ends. Also, frame10B has projection 52B on both ends, and frame10D has projection52D on both ends. Projection52B and 52D, recess54A and 54C engage each other respectively but also detachable. When cell aggregate is dispensed, projection52B and 52D engage to recess 54A and 54C respectively and four connected frame10 will form rectangular shape support1. Later when string member is removed from three-dimensional cellular structure, projection52B and 52D, recess 54A and 54C are detached, consequently makes four frames separable. Also, when four frame10 are separated, connection between string member20 and frame 10 can be released. For example, string member20 pulled out of frame10 fixture, and/or cut by knives.

Fig.5(B) shows other measure to separate frames. This measure is to separate four frames into two parts. Frame10A and 10D remain engaged and frame10B and 10C remain engaged. With this separating method, removal of string member20 from three-dimensional cellular structure existing in x direction and Y direction is still possible.

Nevertheless, separating method of frames are not limited to as described inFig.5. For example, when frame 10 is made with soft and easily cuttable materials, frames can be cut by knives, laser and such at multiple cutting locations to separate one frame from another frame, and also, cutting location can be between frame and string member. In this case, frame 10 can not be reused.

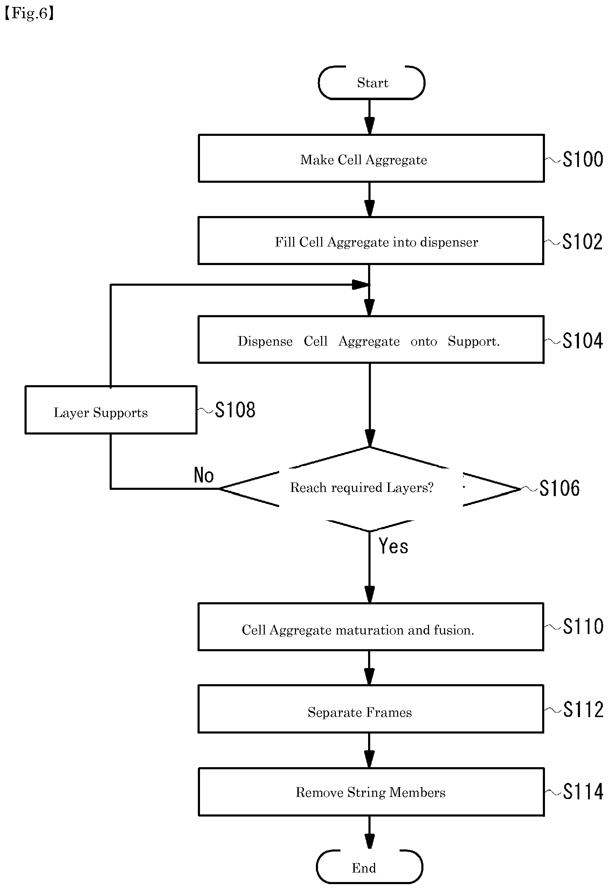

Next, method of three-dimensional cellular structure using support1 is described using Fig. 6. First, make cell aggregate (Spheroid) in range of certain size by aggregating plurality of living cell. (S100). Method of making cell aggregate can be any measure, and one example is described in Patent Document #3.

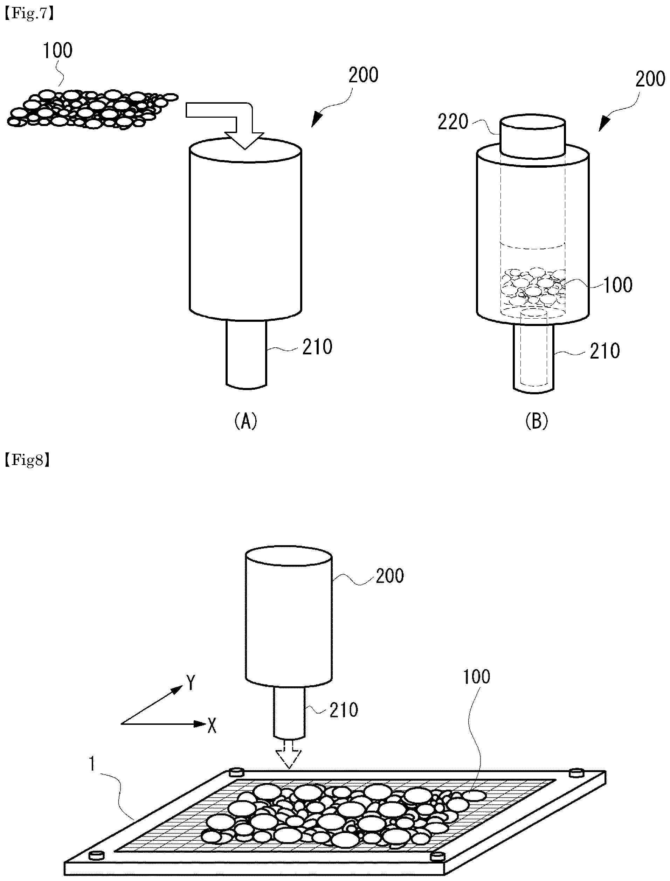

After making cell aggregate, cell aggregate is filled into dispenser (S102). Fig. 7(A) shows one example how cell aggregate is filled into dispenser. Cell aggregate solution100 which contains plurality of cell aggregate is filled into dispenser200. Cell aggregate size contained in cell aggregate solution100 can be various, but minimum size of cell aggregate Dmin is larger than the space Px and Py width which formed by string member20. As shown in Fig.7(B), dispenser200 comprise discharge part210 and by manipulating cylinder220 cell aggregate solution100 can be discharged through discharge part210.

Next, Cell aggregate is dispensed onto support surface (S104). As shown in Fig.8, by scanning to X and Y directions according to desired shape of three-dimensional cellular structure, cell aggregate 100 is dispensed onto string member20. Other way is to fix location of dispenser200, and mount support1 on stage, and move stage 1 to x direction and y direction. The smallest size of cell aggregate Dmin dispensed onto support is larger than Px and Py width and yet dispensed cell aggregate does not go through string member20 and held by string member20. It should be noted that part of sphere shape or orbital shape cell aggregate can be protrude through the space of Px, Py toward the bottom.

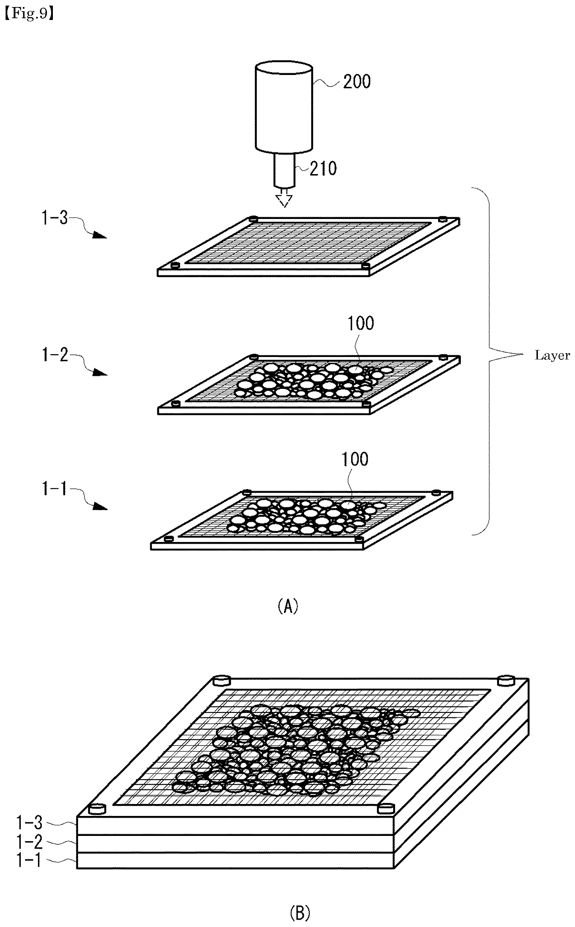

Next, number of layer is monitored (S106). Number of layer is determined by Z axis height of three-dimensional cellular structure to be fabricated. Until given number of layer is completed, additional support is layered (S108) and cell aggregate solution is dispensed onto (S104). When support is layered, each support is aligned by alignment measure30 and 40, Fig.9(A) shows how cell aggregate solution100 is dispensed onto support1-1 and 1-2, after completion of dispensing cell aggregate solution100 onto suport1-2, support1-3 is layered on top of support1-2 and cell aggregate solution100 is dispensed. Thus, multiple supports are layered and cell aggregate solution100 is dispensed depending on the size of three-dimensional cellular structure to be fabricated. Fig.9(B) shows how three support1-1,1-2 and 1-3 are layered. When multiple supports are layered, a part of cell aggregate dispensed on upper support can protrude from net space gap downward and have contact to part of cell aggregate dispensed onto lower support.

When designated number of supports are layered, dispensing cell aggregate solution finishes and they are dipped into medium suitable for cell culture so that cell aggregate fuse together (S110). For example, layered support1 with cell aggregate is dipped in container filled with medium. At that time, certain vibration or shake can be applied to support and/or container. Cell aggregate on support adhere and fuse together with cell aggregate contacting in horizontal direction. Also, cell aggregates adhere and fuse together with cell aggregate on each layered support in vertical direction. As there is no obstacle to block nutrients and oxygen delivery between cell aggregates dispensed on one support, sufficient nutrients and oxygen reach to cell aggregates. Also, as only string member20 lie in between cell aggregates on layered support, cell aggregate has access to nutrients and oxygen through the net shaped space. This condition brings ideal environment for cell adhesion and fusion. Time duration shall be determined depending on cell types.

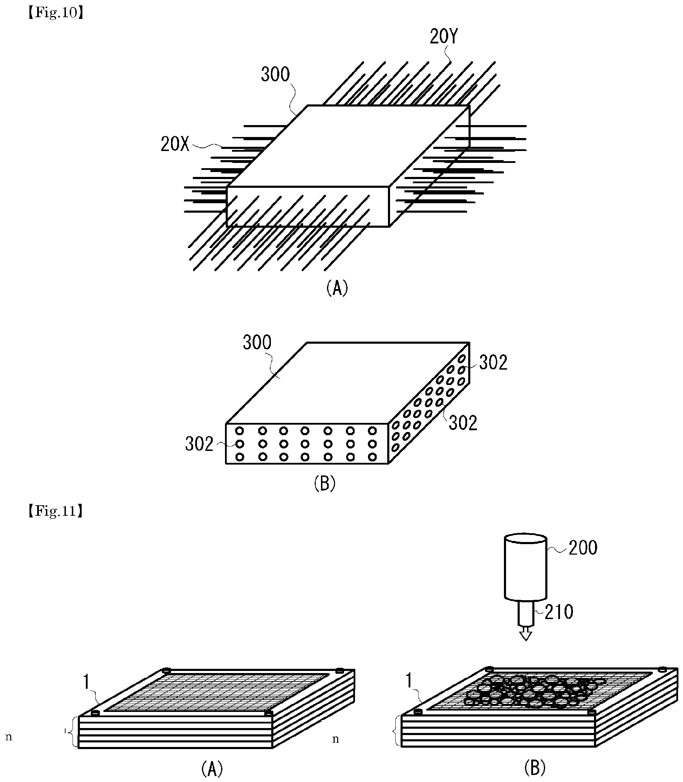

After adhesion and fusion of cell aggregate is completed, frame of layered supports is separated (S112). When separating frames, as of Fig.5(A) and (B), part or whole frame can be separated, or one end of string member20 is separated or cut by knives or laser. Fig10(A) shows schematic diagram of fused cell aggregate, which is three-dimensional cell aggregate300 after all frames are separated. On this schematic diagram, three-dimensional cell aggregate300 is rectangular parallelepiped shape and possess in its inside multiple string member20X which lies in X direction and string member20Y which lies in Y direction, and these string member20X and string member20Y stick out from side of three-dimensional cellular structure.

Next step is removal of string member from three-dimensional cellular structure(S114). Preferably, from three-dimensional cellular structure shown in Fig.10(A), string member20X is removed in X direction, and string member20Y is removed in Y direction, whole string member is completely removed from three-dimensional cellular structure. Fig10(B) is schematic diagram of three-dimensional cellular structure after removal of string member20. On each side of three-dimensional cellular structure, there are small pore302 as the result of removal of string member20. These small pores302 will be closed as cell aggregate adhere and fuse together in some time.

As described as above embodiment, using support which net shaped space is formed inside, dispensing cell aggregate into net shaped space, layer these supports with dispensed cell aggregate, and then removing string member from adhered and/or fused cell aggregate, it is made easier to form plan three-dimensional cellular structure in X and Y direction on one support, and also it is made easier to form thicker three-dimensional cellular structure by layering support in Z direction. Also, cell aggregates are held by very thin diameter string member20, which blockage of nutrients and oxygen is minimal. Furthermore, string members will be removed from three-dimensional cellular structure, obtained three-dimensional cellular structure does not contain any foreign substance and consequently consists of only required cells.

Above embodiment explained one procedure which cell aggregate is dispensed onto one support and then layered multiple supports, but other procedure is possible such as layering only supports with no cell aggregate is dispensed first, and then dispense cell aggregate onto layered supports. Fig.11(A) shows how support1 are layered by n number, and Fig.11(B) shows cell aggregate is dispensed onto from top of support1 by dispenser200. Preferably, when this procedure is done, gap distance of support string member Px and Py shall be decreased from top to the bottom. When gap distance of top support string member is Px_max, Py_max, and gap distance of bottom support string member is Px_min, Py_min, size of cell aggregate held by dispense200 can be any size between Px_min, Py_min ∼ Px_max, Py_max. From these configurations, cell aggregate which pass through top layer string member space shall be caught by lower string member.

Next second embodiment of present invention is explained. In the first embodiment, net shaped space is formed on whole support area. In the second embodiment, net shaped space is formed according to arbitrary shape and size of three-dimensional cellular structure.

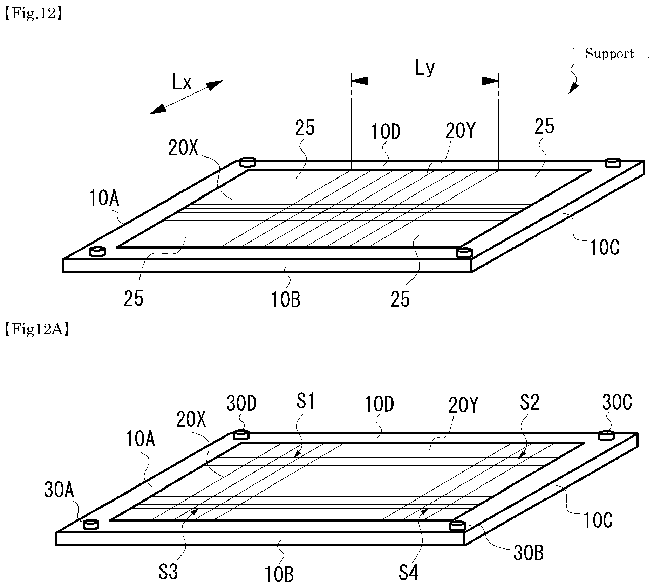

Fig.12 shows schematic diagram of support used for second embodiment. In this embodiment, support1 has string member20x in x direction is located in width Lx, and string member20y in y direction is located in width Ly. From this, support1 has net shaped space only in between width Lx and Ly. Area25 which does not have string member will not hold cell aggregate which is dispensed from dispencer200. Consequently, three-dimensional cellular structure is formed on Width Lx, Ly area.

Above mentioned example used rectangular space Lx, Ly, but space shape can be different. For example, as shown in Fig.12(A), multiple rectangular space S1, S2, S3 and S4 can be formed inside of support. In this case, four three-dimensional cellular structures which have corresponding shape to S1, S2, S3 and S4 are obtained at one process.

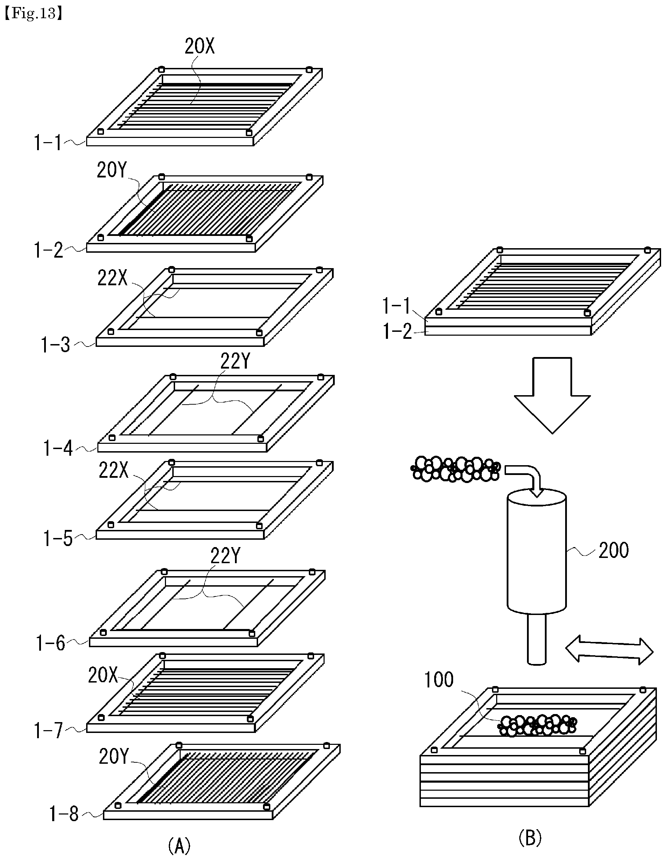

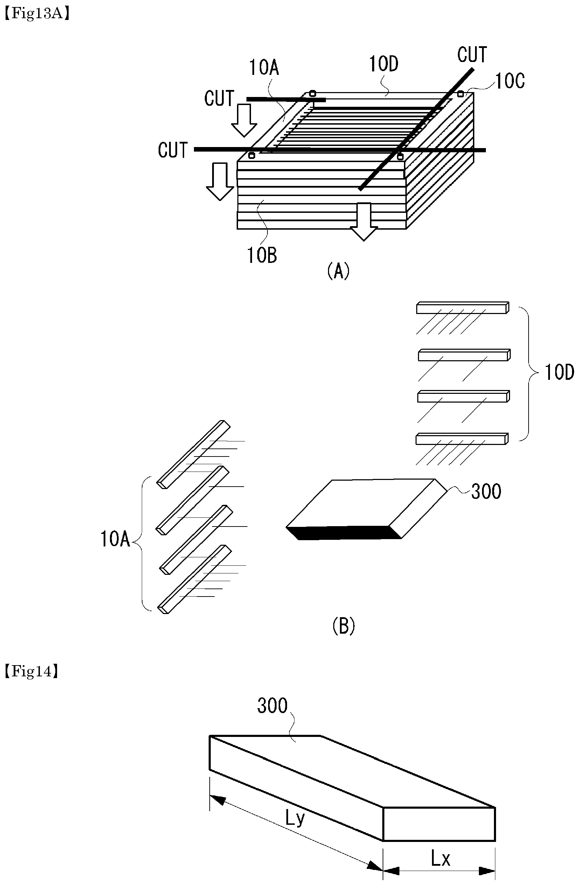

In second embodiment as well as used in first embodiment, multiple support shall be layered according to the height of designed three-dimensional cellular structure. Fig.13 shows how supports are layered and cell aggregate are dispensed in second embodiment. Fig13(A) shows support1-1 and 1-2 make upper lid and supportl-7, 1-8
make bottom lid. Number of Support1-3 ∼1-6 shall be determined by the height of designed three-dimensional cellular structure. Support 1-1,1-7 comprise multiple number of string member20X located in uniform gap distance in X direction, and Supportl-2,1-8 comprise multiple number of string member20Y located in uniform gap distance in Y direction. Supportl-3 comprise a pair of string member22X in x direction which regulate the x direction boundary of three-dimensional cellular structure, and Support 1-4 comprise a pair of string member22Y in y direction which regulate the y direction boundary of three-dimensional cellular structure. Preferably, support 1-3 and 1-4 are layered by each other. Next, as shown on Fig.13(B), cell aggregate is dispensed onto support with Upper Lid1-1, 1-2 detached. After dispensing is completed, upper Lid1-1 1-2 is mounted onto Supportl-3.

Fig13(A) shows one example of how string member is removed. As shown on Fig.13A(A), support is cut at three CUT locations from top to bottom, and separate frame10B and 10C from the body. And then, as shown on Fig.13A(B), move remaining frame10A to X direction, and move remaining frame10D to Y direction so that all string member20 is removed from three-dimensional cellular structure300.

Fig.14 is schematic perspective view of three-dimensional cellular structure fabricated by support described in second embodiment. Three-dimensional cellular structure300 is fabricated at the size of space Lx and Ly which is regulated by string member in X direction and string member in Y direction.

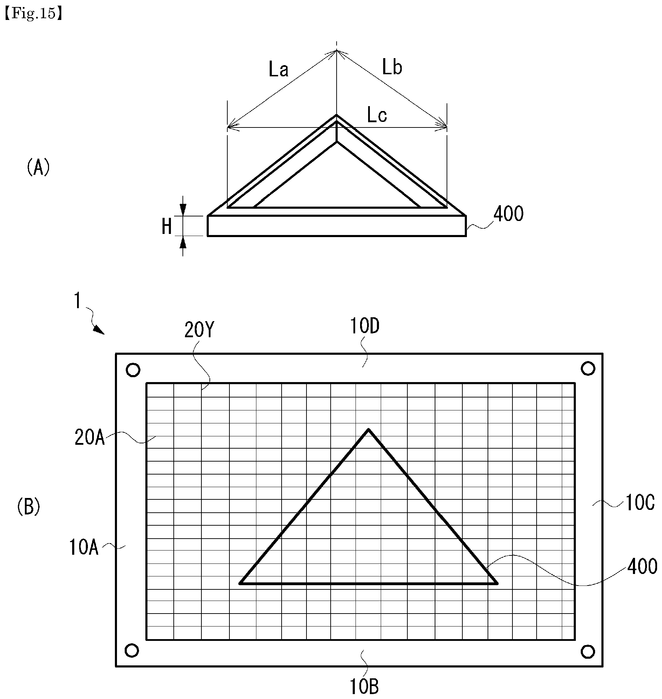

Next, the third embodiment is described. At the third embodiment, guiding member which regulate the shape of cell aggregates to be dispensed into, is provided on a support. Fig.15(A) is an example of guiding member400, and guiding member400 provides triangle shape of lengh La, Lb and Lc. The heightH of guiding member400 also function as spacerΔd between support1 when layered. Guiding member400 is mounted onto support at fixed location as shown in Fig.15(B). Fixing measure can be various way, as one example is to draw marking on support1 and guiding member400 is aligned onto support marking. Guiding member400 can be connected lightly by other fixture or adhesives, or simply placed on the support. Also, preferably guiding member surface s coated with non-adherent material for easy removal from cell aggregate.

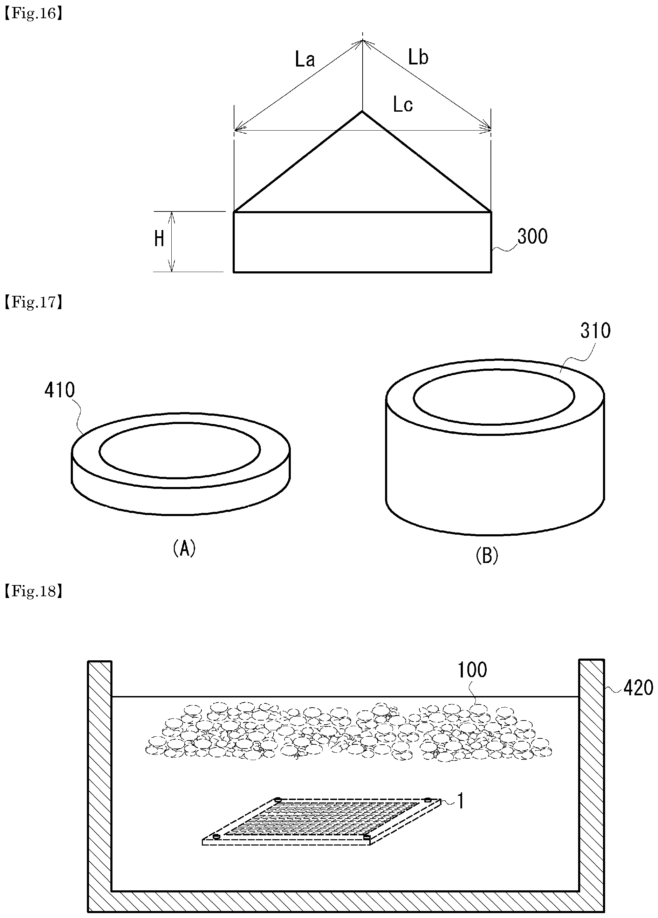

As shown in Fig15(B), dispense200 dispense cell aggregate into the space guided by guiding member400. Next, other support is layered and guiding support400 is also placed, and cell aggregate is dispensed into as well. Consequently as shown in Fig. 16, Three-dimensional cellular structure300 is obtained.

Above example used triangle shape guiding member, and the shape can be others such as circle, donuts, rectangular, oval etc. For example, as shown in Fig.17(A), when donuts shape guiding member41 is used, donuts shaped three-dimensional cellular structure is obtained.

Next, a fourth option is explained. Embodiment 1-3 used dispenser to fill in cell aggregate onto supports, while the fourth option uses process to filter out cell aggregate in solution by support. One example of this is shown in Fig. 18. As shown in this figure, solution containing cell aggregate 100 is held in a container,
support1 filters out cell aggregate from solution. From this procedure, cell aggregate larger than the size of net shaped space of string member20 is filtered out onto string member.

In case supports with uniform size of net shaped space on string member20 are used, the effect is identical to the process of dispensing cell aggregate of the size larger than the net shaped space onto support. Also, it is possible to filter out whole cell aggregate without exception by using supports with different size of net shaped space formed by string member.

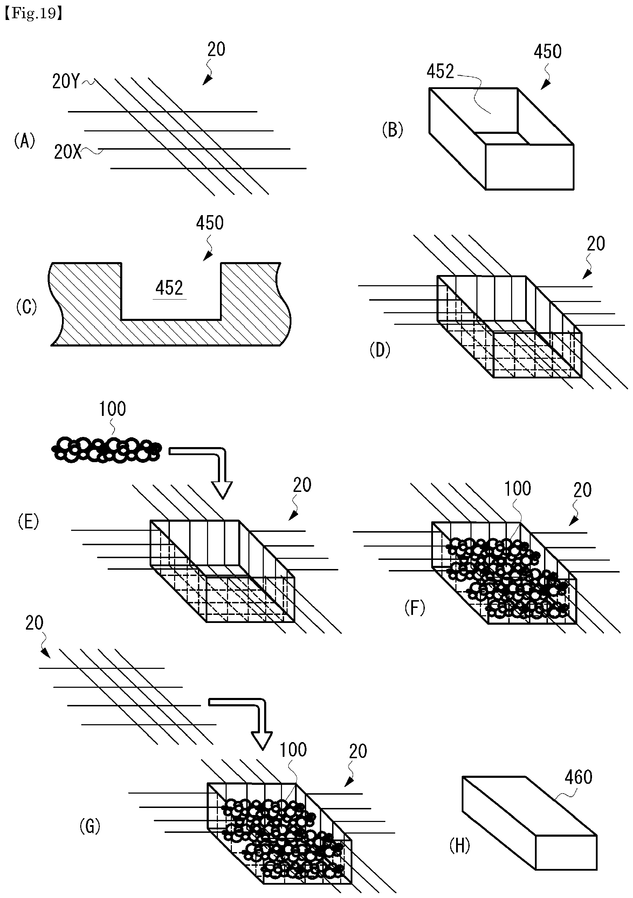

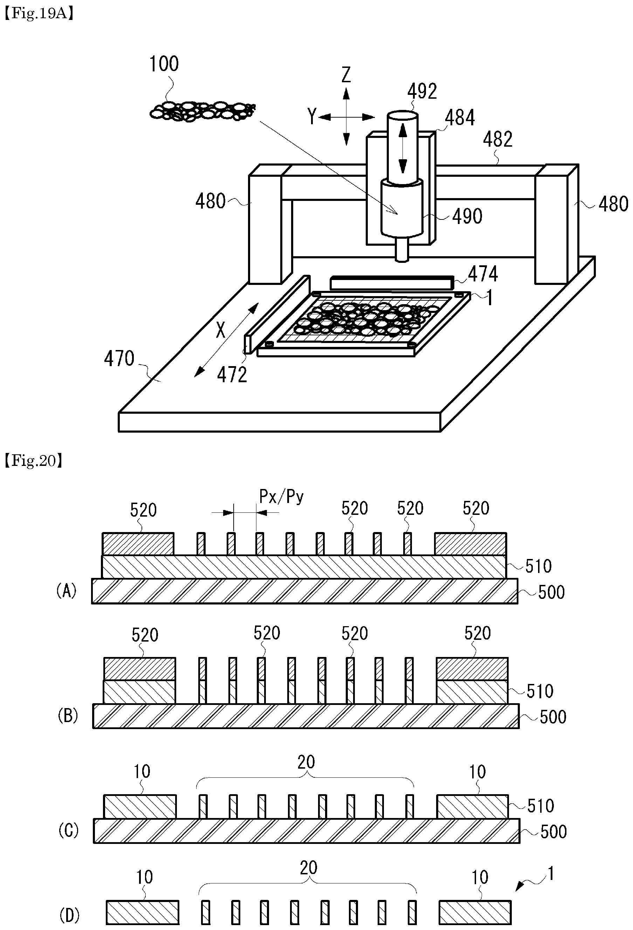

Next, fifth embodiment is explained. The fifth embodiment is to make three-dimensional cellular aggregate by net molding process. Fig.19 shows example of embodiment. Prepare string member which is capable to form net shaped space as shown on Fig.19(A), and prepare mold450 in shape as shown in Fig.19(B), (C). Mold450 can be any shape, and this example has rectangular shape mold452. Mold 450 can be made by various process, and this can be made by plastic injection process. It is also possible to form mold450 by laser processing, punching process. Next, as shown on Fig.19(D) string member20 is formed along the boundary of inner space450. Next, as shown in Fig.19(E), (F), cell aggregate 100 is dispensed into innerspace452 of mold450 by dispenser. Then, as shown on Fig.19(G), lid formed by string member20, and placed onto mold450. This lid prevents cell aggregate from spilling out from mold when shaken inside of solution with medium. Next after cell aggregate fused together, string member20 is lifted up from mold450 and cell aggregate 100 is removed from mold450. Three-dimensional cellular aggregate460 which reflect the shape of mold450 is obtained by removing string member20. Preferably, string member20 is made with non-adherent material, or its surface is coated by non-adherent material, so that removal of string member20 from cell aggregate 100 is easier.

Next, sixth embodiment of present invention is explained. The sixth embodiment is to apply the use of support of present invention to three-dimensional dispenser. As shown on Fig.19A, automated-dispenser comprise stage470, reference guide472 (x direction) and gude474(y direction), a pair of moving tower480 which moves in x direction,
bridging member482 which connect between tower480 in y direction, sliding member484 which is movable to Y and Z direction, dispenser490 and plunger492 attached on sliding member 484.

Dispenser490 will be filled with cell aggregate100, as plunger492 is actuated in Z direction so that cell aggregate100 is extruded from tip of dispenser490. Automated dispenser is pre-programmed of the shape of support1 and the initial filling point of cell aggregate, and automated dispenser scan in X,Y,Z direction according to the three-dimensional data of three-dimensional cellular structure to be made. After support1 is positioned according to reference gude472 and 474, dispencer490 scan in direction of X and Y, simultaneously plunger492 is actuated, cell aggregate is extruded onto support from tip of dispencer490. When one support dispensing is completed, another support is layered and again cell aggregate is dispensed according to three-dimensional data. This embodiment enable fast dispensing of cell aggregate onto supports.

Next, several ways to fabricate support are explained. Support1 comprise, for example, rectangular frame10. Support1 can be any shape and size, and space surrounded by frame10 is further divided into smaller spaces by string member20. String Member20 consists of thread or needle shaped member and end of string member20 is adhered or fixed in other way to frame.

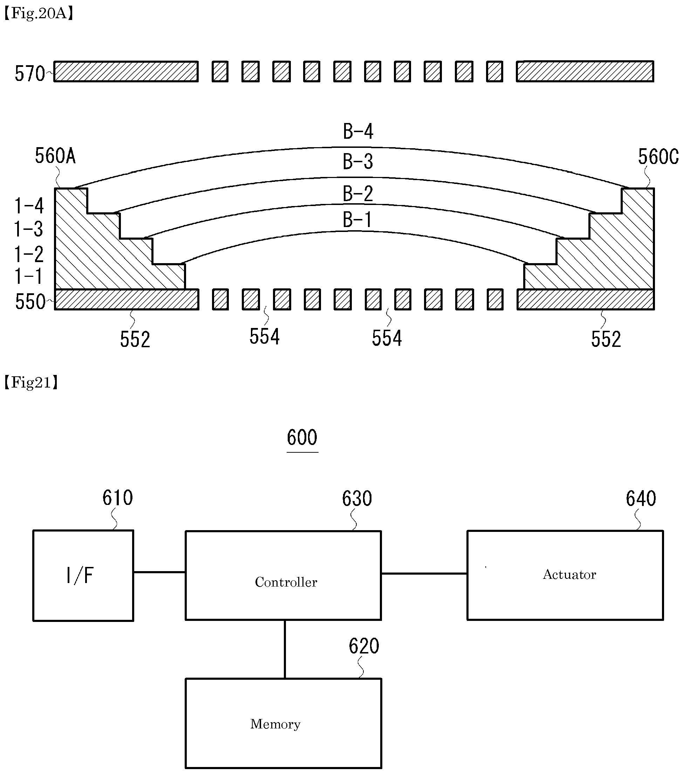

Other way to fabricate support is to use semiconductor lithography process to form multiple spaces onto thin plastic or metal material. Fig.20 shows schematic cross- sectional view of the process. As shown in Fig.20(A), place thin material510 which will become support onto resin film500 such as UV cured type, and patterned musk520 is formed by photo-lithography process, Musk520 is designed so that gap distance Px, Py form matrix shape. Next Fig.20(B) shows exposed area of thin material510 are removed by etching process. Anisotropic etching process is preferred for accuracy. Etching process can be either wet or dry process. Next, as shown in Fig.20(C), musk520 is removed and then as shown in Fig.20(D) resin film500 is removed by exposing to UV light. By these processes, integrated support1 consists of frame10 and string member20 as one is fabricated. Above mentioned example used photo lithography process, but musk520 fabrication method can be other method such as laser processing.

Furthermore as other way of fabrication, cavity with gap distance Px, Py can be formed by laser processing onto thin material to form support. Also, punching process can be used to form cavity onto thin material by applying embossing member with Px, Py shaped pattern. Also, 3D printer can be used to from integrated support with frame and string member as one by resin materials.

Other way to fabricate support, string member20 of support can be formed by bonding wire. Fig.20A is schematic cross-sectional view when string member is formed by bonding wires. As preferred example, base550 is prepared first. Base550 is made with metal material such as stainless steel. Base520 comprise outer frame552 and center net area which comprise multiple opening524. As an example, opening554 can be made by etching process on base550. On outer frame552, frame560A and 560C with level step in its inside are formed. Frame560A and 560C are equivalent to frame10A, 10C of Fig. 1, respectively. On this example. Frame560A and 560C possess 4 level steps equivalent to 4 layers of supports. On Layel-1, bonding wireB-1 runs in X and Y directions, and on layel-2 bonding wireB-2 runs in X and Y directions, on layel-3 bonding wireB-3 runs in X and Y directions, on layel-4, bonding wireB-4 runs in X and Y directions. This structure is practically same structure as 5 supports are layered.

To be noted, gap distance of bonding wireB-1 ∼B-4 in X and Y direction does not have to be uniform. Similarly gap distance of opening554 in X and Y direction does not have to be uniform. The gap distance can be various as explained in Fig.11. Furthermore if necessary, top lid570 can be used. This top lid can be same as base550.

Fig.21 is block chart of electrical structure of equipment used for three-dimensional cellular structure fabrication. Equipment600 comprise interface610 which enable input from operator and connection between equipment and peripheral equipment, memory620 which stores data and program, controller630, and actuator640 which drives dispenser200. Memory620 comprise program for fabrication process to fabricate three-dimensional cellular structure, contoroller630 conduct said program to fabricate three-dimensional cellular structure. As a preferable example, memory620 stores 3D data of three-dimensional cellular structure, controller630 controls scanning track of dispenser200. This means that the dispenser provides the shape of cell aggregates on the support based on 3D data. In this case, the shape of each layer of support are determined by 3D data. For example, when 3D data is corn shape, dispenser200 deliver cell aggregate in different shapes, which is smaller and smaller, on each layer. Also, actuator640 moves stage to X and Y direction to dispense cell aggregate onto supports.

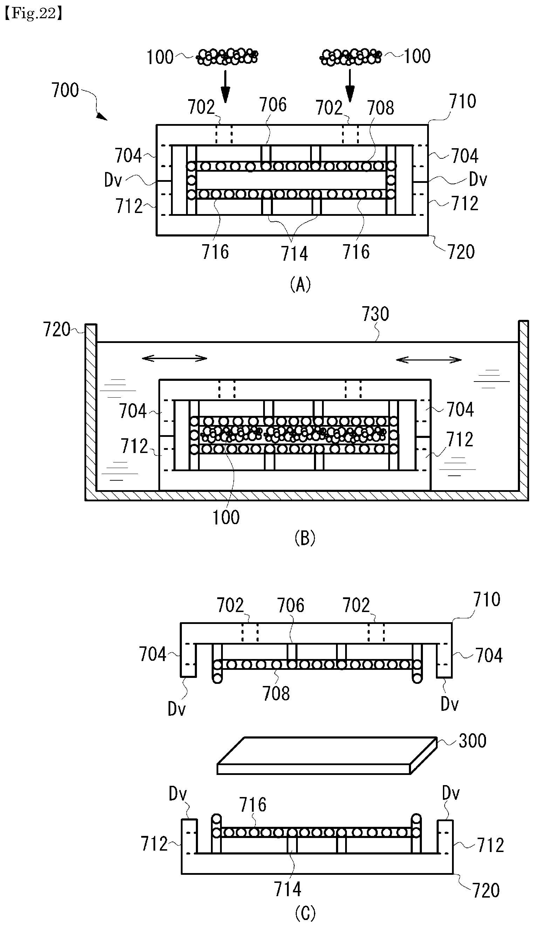

Next, the seventh embodiment is described. Fig.22 shows how 3D support is made by 3D Printer. In this embodiment, this 3D support is called as mold700. Mold700 is made with non-toxic material to human being. Preferably, mold700 is made with material such as polycarbonate which has high melting temperature for high temperature sterilization. Also preferably, mold700 is coated with non-adherent material to prevent cell adhesion to mold.

Mold700 comprise, as shown in Fig.22(A), upper part710 and lower part720. Upper part710 and lower part720 possess net shaped area where cell aggregates are held and they are made by 3D printer. When cell aggregate is filled in mold and cell aggregate is fused, in order to remove three-dimensional cellular structure from mold 700, mold700 is separated to upper part710 and lower part720 at cutting positionDv.

Upper part710 comprise top and side member, multiple filling hole702 connected inner space on top member. Also, side of upper part710 has multiple circulation hole704 for medium flow. Upper part710 comprise column support706 which extend from top, and multiple support column706 are connected to upper net shaped member708 which form three-dimensional space. Lower part720 comprise column support714 which extend from bottom, and multiple support column714 are connected to lower net shaped member716 which form three-dimensional space
Cell aggregate100 is filled in through filling hole702. One preferable example, the gap distance of upper net shaped member708 is larger than the size of cell aggregate but the gap distance of lower net shaped member716 is smaller than the size of cell aggregate so that cell aggregate pass through upper net shaped member708 and trapped by lower net shaped member716. It needed to be noted that this is one example and gap distance of upper net shaped member708 and lower net shaped member can be the same.

Next, as shown in Fig.22(B), mold700 with cell aggregates filled is soaked in container720 filled with medium730. By applying movement by shaker, medium730 shall circulate through filling hole702, circulating hole704 and 714, which enable effective culture of cells. When maturation is completed, as shown in Fig.22(C), mold700 is separated to upper par710 and lower part720, and three-dimensional cellular structure is obtained.

String member20 used by embodiment 1-6 are made of either adherent or non-adherent materials. Preferably, string member20 is made with non-adherent material for easy removal, coated with non-adherent material otherwise.

Cell aggregate used by embodiment1-3 are consists of either single cell type or multiple cell types. When multiple cell types are used, obtained three-dimensional cellular structure comprise multiple cell type.

Three-dimensional cellular structure made by enbodiment1-5 are used for medical and research purpose, as well as potential application of joint regeneration, toxicity screening, cancer research, neural regeneration, cardiovascular regeneration, as such.

Above, many embodiments are described, but should be noted that present invention is not limited to these embodiments. Rather, the scope of the present invention is defined by the appended claims.

## Claims

1. A method of fabricating a cellular structure, the method comprising:
a step of feeding a cell aggregate onto thread or needle shaped members,
said member forming a net shaped space by said thread or needle shaped member,
a step of layering a support with a feed cell aggregate; and
a step of removing said threads or needles from a fused cellular structure;
wherein the support comprises;
multiple frames; and
multiple thread or needle shaped members which form a net shaped space surrounded by the multiple frames; wherein
said net shaped space can hold cell aggregates.

2. A method of fabricating a cellular structure, the method comprising:
a step of layering supports having a net shaped space formed by thread or
needle shaped members,
a step of feeding cell aggregate onto the said layered support; and
a step of removing said thread or needle shape members from a fused cellular structure;
wherein the support comprises;
multiple frames; and
multiple thread or needle shaped members which form a net shaped space surrounded by the multiple frames; wherein
said net shaped space can hold cell aggregates.

3. A method of fabricating a cellular structure, the method comprising:
a step of layering supports, said support having a thread or needle shaped member, said member provide a boundary of the arbitrary cellular structure shape;
a step of feeding a cell aggregate onto support; and
a step of removing said threads or needles from a fused cellular structure;
wherein the support comprises;
multiple frames; and
multiple thread or needle shaped members which form a net shaped space surrounded by the multiple frames; wherein
said net shaped space can hold cell aggregates.

4. The method of any of claims 1-3, comprising loading cell aggregates into a dispenser, scanning said dispenser and dispensing said cell aggregate onto the thread or needle member.

5. The method of any of claims 1-2, comprising placing a guiding member which provides a shape of cellular structure, wherein the cell aggregate is fed into said guide member.

6. The method of any of claims 1-5, comprising immersing the support with the cell aggregate already fed on top into liquid containing nutrients, and applying vibration to the support and/or container.

7. The method of any of claims 1-6, wherein said removing process comprises removing the thread or needle shaped member from said cell aggregate in the X direction or Y direction.

8. The method of claim 1, wherein the support possesses a net shaped space smaller than the size of the cell aggregate and net shaped space larger than the size of cell aggregate, wherein the boundary between said smaller net shaped space and larger net shaped space provides the shape of cell aggregate to be fused.

9. A support used for a three-dimensional cellular structure comprising;
multiple frames; and
multiple thread or needle shaped members which form a net shaped space surrounded by the multiple frames; wherein
said net shaped space can hold cell aggregates.

10. A support used for a three-dimensional cellular structure comprising;
multiple frames; and
multiple thread or needle shaped members which provide a boundary to an arbitrary cellular structure shape, said members located within the space surrounded by the multiple frames; wherein
a net shaped space is provided by the thread or needle shaped members, and wherein said net shaped space can hold cell aggregates.

11. The support of claim 9, wherein said support comprises a guiding member which provides the shape of three-dimensional cellular structure, said guiding member located inside of net shaped space.

12. The support of claim 9, wherein said support possesses a net shaped space smaller than the size of the cell aggregate and a net shaped space larger than the size of the cell aggregate, wherein the boundary between said smaller net shaped space and larger net shaped space provides the shape of cell aggregate to be fused.

13. The support of any of claims 9-12, wherein said thread or needle shaped members are made of non-adhesive materials against the cell aggregate.

14. The support of any of claims 9-12, wherein said thread or needle shaped members are non-adhesive coated against cell aggregate.

15. A method of fabricating a cellular structure utilizing automated dispensing equipment, the method comprising:
a step of preparing a support comprising multiple frames and multiple thread or needle shaped members which form net shaped space surrounded by the multiple frames, wherein said net shaped space can hold cell aggregates;
a step of holding cell aggregates being fed from said automated dispensing equipment within said net shaped space; and
a step of removing thread or needle shaped member from a fused cell aggregate.

## Patentansprüche

1. Verfahren zur Herstellung einer zellulären Struktur, wobei das Verfahren Folgendes umfasst:
einen Schritt des Zuführens eines Zellaggregats auf faden- oder nadelförmige Elemente, wobei das Element einen netzförmigen Raum durch das faden- oder nadelförmige Element bildet,
einen Schritt des Schichtens eines Trägers mit einem Zuführungszellaggregat; und
einen Schritt des Entfernens der Fäden oder Nadeln aus einer fusionierten zellulären Struktur;
wobei der Träger Folgendes umfasst:
mehrere Rahmen; und
mehrere faden- oder nadelförmige Elemente, die einen netzförmigen Raum bilden, der von den mehreren Rahmen umgeben ist; wobei
der netzförmige Raum Zellaggregate halten kann.

2. Verfahren zur Herstellung einer zellulären Struktur, wobei das Verfahren Folgendes umfasst:
einen Schritt des Schichtens von Trägern, die einen netzförmigen Raum aufweisen, der durch faden- oder nadelförmige Elemente gebildet wird;
einen Schritt des Zuführens von Zellaggregat auf den geschichteten Träger; und
einen Schritt des Entfernens der faden- oder nadelförmigen Elemente aus einer fusionierten zellulären Struktur;
wobei der Träger Folgendes umfasst:
mehrere Rahmen; und
mehrere faden- oder nadelförmige Elemente, die einen netzförmigen Raum bilden, der von den mehreren Rahmen umgeben ist; wobei
der netzförmige Raum Zellaggregate halten kann.

3. Verfahren zur Herstellung einer zellulären Struktur, wobei das Verfahren Folgendes umfasst:
einen Schritt des Schichtens von Trägern, wobei der Träger ein faden- oder nadelförmiges Element aufweist, wobei das Element eine Abgrenzung der beliebigen zellulären Strukturform bereitstellt;
einen Schritt des Zuführens eines Zellaggregats auf den Träger; und
einen Schritt des Entfernens der Fäden oder Nadeln aus einer fusionierten zellulären Struktur;
wobei der Träger Folgendes umfasst:
mehrere Rahmen; und
mehrere faden- oder nadelförmige Elemente, die einen netzförmigen Raum bilden, der von den mehreren Rahmen umgeben ist; wobei
der netzförmige Raum Zellaggregate halten kann.

4. Verfahren nach einem der Ansprüche 1-3, das das Beladen von Zellaggregaten in einen Verteiler, Scannen des Verteilers und Verteilen des Zellaggregats auf das faden- oder nadelförmige Element umfasst.

5. Verfahren nach einem der Ansprüche 1-2, das das Positionieren eines Führungselements umfasst, das eine Form der zellulären Struktur bereitstellt, wobei das Zellaggregat in das Führungselement zugeführt wird.

6. Verfahren nach einem der Ansprüche 1-5, das das Eintauchen des Trägers mit dem bereits obenauf zugeführten Zellaggregat in Flüssigkeit, die Nährstoffe enthält, und das Anwenden von Vibration auf den Träger und/oder Behälter umfasst.

7. Verfahren nach einem der Ansprüche 1-6, wobei der Entfernungsprozess das Entfernen des faden- oder nadelförmigen Elements aus dem Zellaggregat in der X-Richtung oder Y-Richtung umfasst.

8. Verfahren nach Anspruch 1, wobei der Träger einen netzförmigen Raum, der kleiner ist als die Größe des Zellaggregats, und einen netzförmigen Raum, der größer ist als die Größe des Zellaggregats, aufweist, wobei die Abgrenzung zwischen dem kleineren netzförmigen Raum und größeren netzförmigen Raum die Form von zu fusionierendem Zellaggregat bereitstellt.

9. Träger, der für eine dreidimensionale zelluläre Struktur verwendet wird, der Folgendes umfasst:
mehrere Rahmen; und
mehrere faden- oder nadelförmige Elemente, die einen netzförmigen Raum bilden, der von den mehreren Rahmen umgeben ist; wobei
der netzförmige Raum Zellaggregate halten kann.

10. Träger, der für eine dreidimensionale zelluläre Struktur verwendet wird, der Folgendes umfasst:
mehrere Rahmen; und
mehrere faden- oder nadelförmige Elemente, die eine Abgrenzung einer beliebigen zellulären Strukturform bereitstellen, wobei sich die Elemente innerhalb des Raums befinden, der von den mehreren Rahmen umgeben ist; wobei ein netzförmiger Raum durch die faden- oder nadelförmigen Elemente bereitgestellt wird und wobei der netzförmige Raum Zellaggregate halten kann.

11. Träger nach Anspruch 9, wobei der Träger ein Führungselement umfasst, das die Form von dreidimensionaler zellulärer Struktur bereitstellt, wobei sich das Führungselement innerhalb des netzförmigen Raums befindet.

12. Träger nach Anspruch 9, wobei der Träger einen netzförmigen Raum, der kleiner ist als die Größe des Zellaggregats, und einen netzförmigen Raum, der größer ist als die Größe des Zellaggregats, aufweist, wobei die Abgrenzung zwischen dem kleineren netzförmigen Raum und größeren netzförmigen Raum die Form von zu fusionierendem Zellaggregat bereitstellt.

13. Träger nach einem der Ansprüche 9-12, wobei die faden- oder nadelförmigen Elemente aus für das Zellaggregat nichthaftenden Materialien hergestellt sind.

14. Träger nach einem der Ansprüche 9-12, wobei die faden- oder nadelförmigen Elemente für Zellaggregat nichthaftend beschichtet sind.

15. Verfahren zur Herstellung einer zellulären Struktur unter Verwendung von automatisierter Verteilervorrichtung, wobei das Verfahren Folgendes umfasst:
einen Schritt des Herstellens eines Trägers, der mehrere Rahmen und mehrere faden- oder nadelförmige Elemente umfasst, die einen netzförmigen Raum bilden, der von den mehreren Rahmen umgeben ist, wobei der netzförmige Raum Zellaggregate halten kann;
einen Schritt des Haltens von Zellaggregaten, die aus der automatisierten Verteilervorrichtung innerhalb des netzförmigen Raums zugeführt werden; und
einen Schritt des Entfernens von faden- oder nadelförmigem Element aus einem fusionierten Zellaggregat.

## Revendications

1. Procédé de fabrication d'une structure cellulaire, le procédé comprenant :
une étape d'alimentation d'un agrégat cellulaire sur des éléments en forme de fil ou d'aiguille, ledit élément formant un espace en forme de filet par ledit élément en forme de fil ou d'aiguille,
une étape de superposition d'un support avec un agrégat cellulaire d'alimentation ; et
une étape de suppression desdits fils ou desdites aiguilles à partir d'une structure cellulaire fusionnée ;
dans lequel le support comprend :
de nombreux cadres ; et
de nombreux éléments en forme de fil ou d'aiguille qui forment un espace en forme de filet entouré par les nombreux cadres ; dans lequel
ledit espace en forme de filet peut maintenir les agrégats cellulaires.

2. Procédé de fabrication d'une structure cellulaire, le procédé comprenant :
une étape de superposition de supports ayant un espace en forme de filet formé par les éléments en forme de fil ou d'aiguille,
une étape d'alimentation d'un agrégat cellulaire sur ledit support en couches ; et
une étape de suppression desdits éléments en forme de fil ou d'aiguille à partir d'une structure cellulaire fusionnée ;
dans lequel le support comprend :
de nombreux cadres ; et
de nombreux éléments en forme de fil ou d'aiguille qui forment un espace en forme de filet entouré par les nombreux cadres ; dans lequel
ledit espace en forme de filet peut maintenir les agrégats cellulaires.

3. Procédé de fabrication d'une structure cellulaire, le procédé comprenant :
une étape de superposition de supports, ledit support ayant un élément en forme de fil ou d'aiguille, ledit élément forme une limite de la forme de structure cellulaire arbitraire ;
une étape d'alimentation d'un agrégat cellulaire sur le support ; et
une étape de suppression desdits fils ou desdites aiguilles à partir d'une structure cellulaire fusionnée ;
dans lequel le support comprend :
de nombreux cadres ; et
de nombreux éléments en forme de fil ou d'aiguille qui forment un espace en forme de filet entouré par les nombreux cadres ; dans lequel
ledit espace en forme de filet peut maintenir les agrégats cellulaires.

4. Procédé selon l'une quelconque des revendications 1 à 3, comprenant le chargement d'agrégats cellulaires dans un distributeur, le balayage dudit distributeur et la distribution dudit agrégat cellulaire sur l'élément de fil ou d'aiguille.

5. Procédé selon l'une quelconque des revendications 1 à 2, comprenant le positionnement d'un élément de guidage qui fournit une forme de structure cellulaire, dans lequel l'agrégat cellulaire est alimenté dans ledit élément de guidage.

6. Procédé selon l'une quelconque des revendications 1 à 5, comprenant l'immersion du support avec l'agrégat cellulaire déjà alimenté sur la partie supérieure dans un liquide contentant des nutriments, et l'application de vibration au support et/ou récipient.

7. Procédé selon l'une quelconque des revendications 1 à 6, dans lequel ledit processus de suppression comprend la suppression de l'élément en forme de fil ou d'aiguille dudit agrégat cellulaire dans la direction X ou la direction Y.

8. Procédé selon la revendication 1, dans lequel le support possède un espace en forme de filet inférieur à la taille de l'agrégat cellulaire et un espace en forme de filet supérieur à la taille de l'agrégat cellulaire, dans lequel la limite entre ledit plus petit espace en forme de filet et ledit plus grand espace en forme de filet apporte la forme de l'agrégat cellulaire devant être fusionné.

9. Support utilisé pour une structure cellulaire tridimensionnelle comprenant :
de nombreux cadres ; et
de nombreux éléments en forme de fil ou d'aiguille qui forment un espace en forme de filet entouré par les nombreux cadres ; dans lequel
ledit espace en forme de filet peut maintenir les agrégats cellulaires.

10. Support utilisé pour une structure cellulaire tridimensionnelle comprenant :
de nombreux cadres ; et
de nombreux éléments en forme de fil ou d'aiguille qui fournissent une limite à une forme de structure cellulaire arbitraire, lesdits éléments étant situés à l'intérieur de l'espace entouré par les nombreux cadres ; dans lequel
un espace en forme de filet est fourni par les éléments en forme de fil ou d'aiguille, et dans lequel ledit espace en forme de filet peut maintenir les agrégats cellulaires.

11. Support selon la revendication 9, dans lequel ledit support comprend un élément de guidage qui fournit la forme de la structure cellulaire tridimensionnelle, ledit élément de guidage étant situé à l'intérieur de l'espace en forme de filet.

12. Support selon la revendication 9, dans lequel ledit support possède un espace en forme de filet inférieur à la taille de l'agrégat cellulaire et un espace en forme de filet supérieur à la taille de l'agrégat cellulaire, dans lequel la limite entre ledit plus petit espace en forme de filet et ledit plus grand espace en forme de filet apporte la forme de l'agrégat cellulaire devant être fusionné.

13. Support selon l'une quelconque des revendications 9 à 12, dans lequel lesdits éléments en forme de fil ou d'aiguille sont constitués de matériaux non adhésifs contre l'agrégat cellulaire.

14. Support selon l'une quelconque des revendications 9 à 12, dans lequel lesdits éléments en forme de fil ou d'aiguille sont revêtus de manière non adhésive contre l'agrégat cellulaire.

15. Procédé de fabrication d'une structure cellulaire utilisant un équipement de distribution automatisée, le procédé comprenant :
une étape de préparation d'un support comprenant de nombreux cadres et de nombreux éléments en forme de fil ou d'aiguille qui forment un espace en forme de filet entouré par les nombreux cadres, dans lequel ledit espace en forme de filet peut maintenir des agrégats cellulaires ;
une étape de maintien d'agrégats cellulaires étant alimentés à partir dudit équipement de distribution automatisée à l'intérieur dudit espace en forme de filet ; et
une étape de suppression d'éléments en forme de fil ou d'aiguille à partir d'un agrégat cellulaire fusionné.
